(19) Europäisches Patentamt
European Patent Office
Office européen des brevets

(11) Veröffentlichungsnummer: **0 259 737 B1**

(12) **EUROPÄISCHE PATENTSCHRIFT**

(45) Veröffentlichungstag der Patentschrift: **25.11.92**

(51) Int. Cl.5: **C07D 261/14**, C07D 239/42, C07D 307/52, C07D 271/06, C07D 213/40, C07D 277/28, C07D 285/12, C07D 235/14, C07D 333/20, C07D 215/12, C07D 277/64

(21) Anmeldenummer: **87112652.0**

(22) Anmeldetag: **31.08.87**

Die Akte enthält technische Angaben, die nach dem Eingang der Anmeldung eingereicht wurden und die nicht in dieser Patentschrift enthalten sind.

# (54) 2-Cyano-2-alkoximino-acetamide.

(30) Priorität: **10.09.86 DE 3630732**

(43) Veröffentlichungstag der Anmeldung: **16.03.88 Patentblatt 88/11**

(45) Bekanntmachung des Hinweises auf die Patenterteilung: **25.11.92 Patentblatt 92/48**

(84) Benannte Vertragsstaaten: **AT BE CH DE FR GB GR IT LI NL**

(56) Entgegenhaltungen:
**EP-A- 0 075 821** **EP-A- 0 088 325**
**EP-A- 0 201 999** **EP-A- 0 206 004**
**EP-A- 0 250 744** **DE-A- 2 312 956**
**US-A- 3 954 992** **US-A- 3 957 847**

**CHEMICAL ABSTRACTS, Band 70, Nr.12, 24.März 1969, Seite 321, Nr.57388g; & ZA 6 706 704.**

(73) Patentinhaber: **BAYER AG**
**W-5090 Leverkusen 1 Bayerwerk(DE)**

(72) Erfinder: **Gayer, Herbert, Dr.**
**Alfred-Delp-Strasse 4**
**W-4019 Monheim(DE)**
Erfinder: **Jelich, Klaus, Dr.**
**Pahlkestrasse 5**
**W-5600 Wuppertal 1(DE)**
Erfinder: **Lunkenheimer, Winfried, Dr.**
**Bismarckstrasse 29**
**W-5600 Wuppertal 1(DE)**
Erfinder: **Brandes, Wilhelm, Dr.**
**Eichendorffstrasse 3**
**W-5653 Leichlingen(DE)**
Erfinder: **Hänssler, Gerd, Dr.**
**Am Arenzberg 58a**
**W-5090 Leverkusen 3(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die Erfindung betrifft neue 2-Cyano-2-alkoximino-acetamide, ein Verfahren zu ihrer Herstellung und ihre Verwendung als Schädlingsbekämpfungsmittel.

Es ist bereits bekannt, daß organische Säurederivate, wie beispielsweise das Zink-ethylen-1,2-bis-dithiocarbamat fungizide Eigenschaften besitzen (vgl. z.B. K.H. Büchel "Pflanzenschutz und Schädlingbekämpfung" S. 137, G. Thieme Verlag, Stuttgart 1977).

Die Wirksamkeit dieser Verbindung ist jedoch insbesondere bei niedrigen Aufwandmengen und Konzentrationen nicht in allen Anwendungsbereichen völlig zufriedenstellend.

Weiterhin sind 2-Cyano-2-alkoximino-N-alkyl-acetamide und 2-Cyano-2-alkoximino-N-alkylaminocarbonyl-acetamide als Fungizide bekannt (vgl. DE-OS 2 312 956, US-3 954 922 und Chem. Abstr. 70 : 57 388g (1969).

Desweiteren sind 2-Cyano-2-oximino-acetamide aus der EP-A-201 999 bekannt.

Es wurden neue 2-Cyano-2-alkoximino-acetamide der allgemeinen Formel (I),

$R^1$ für jeweils geradkettiges oder verzweigtes, gegebenenfalls durch Phenyl substituiertes Hydroxyalkyl oder Hydroxyalkoxyalkyl mit jeweils 1 bis 4 Kohlenstoffatomen in den einzelnen Alkylteilen steht, außerdem für jeweils gegebenenfalls ein- bis dreifach, gleich oder verschieden im Arylteil substituiertes Heteroaryl oder Heteroarylalkyl mit 1 bis 4 Kohlenstoffatomen im geradkettigen oder verzweigten Alkylteil steht, wobei als Heteroarylrest jeweils vorkommen kann: Pyridyl, Pyrimidyl, Triazinyl, Chinolyl, Isochinolyl, Oxazolyl, Isoxazolyl, Oxadiazolyl, Benzoxazolyl, Thiazolyl, Isothiazolyl, Thiadiazolyl, Benzthiazolyl, Imidazolyl, Benzimidazolyl, Pyrrolyl, Furanyl, Thienyl, Pyrazolyl oder Triazolyl und wobei als Substituenten im Heteroarylteil oder im benzannellierten Ring jeweils infrage kommen: Hydroxy, Fluor, Chlor, Brom, Cyano, Methyl,Ethyl, n-oder i-Propyl, n-, i-, s- oder t-Butyl, Allyl, Butenyl, Methoxy, Ethoxy, Methylthio, Benzyl, Phenyl, Acetyl, Propionyl, Methoxycarbonyl, Ethoxycarbonyl, Methylaminocarbonyl oder Dimethylaminocarbonyl und

$R^2$ für gegebenenfalls ein- bis dreifach, gleich oder verschieden substituiertes, geradkettiges oder verzweigtes Alkyl mit 1 bis 12 Kohlenstoffatomen steht, wobei als Substituenten genannt seien: Cyano, Acetyl, Propionyl, Methoxycarbonyl, Ethoxycarbonyl, Methylcarbonyloxy, Ethylcarbonyloxy, gegebenenfalls ein- bis dreifach, gleich oder verschieden durch Fluor, Chlor, Brom, Methyl und/oder Ethyl substituiertes Phenyl;
außerdem für jeweils geradkettiges oder verzweigtes Alkenyl oder Halogenalkenyl mit 3 bis 6 Kohlenstoffatomen und gegebenenfalls 1 bis 3 Halogenatomen oder für geradkettiges oder verzweigtes Alkinyl mit 3 bis 6 Kohlenstoffatomen steht,
ausgenommen die Verbindungen 2-(2-Cyano-2-methoximino-acetamido)-thiazol,3-Methyl-5-(2-cyano-2-methoximino-acetamido)-isooxazol und 2-Methyl-5-(2-cyano-2-methoximino-acetamido)-1,3,4-thiadiazol, gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen 2-Cyano-2-alkoximino-acetamide der allgemeinen Formel (I),

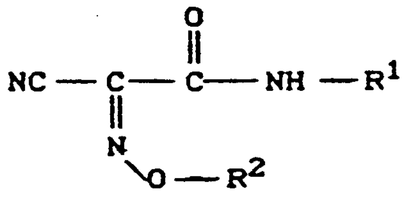

(I)

in welcher

$R^1$ und $R^2$ die oben angegebene Bedeutung haben,

erhält, wenn man 2-Cyano-2-oximino-acetamide der Formel (II),

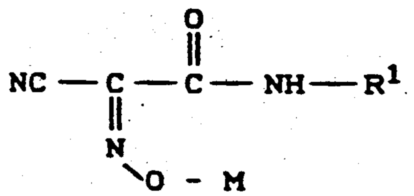

(II)

in welcher

- M für Wasserstoff oder für ein Alkalimetallkation steht und
- $R^1$ die oben angegebene Bedeutung hat,

mit Alkylierungsmitteln der Formel (III),

$R^2$-A (III)

in welcher

- A für eine geeignete Abgangsgruppe steht und
- $R^2$ die oben angegebene Bedeutung hat,

gegebenenfalls in Gegenwart eines Verdünnungsmittels und gegebenenfalls in Gegenwart eines Säurebindemittels umsetzt.

Schließlich wurde gefunden, daß die neuen 2-Cyano-2-alkoximino-acetamide der allgemeinen Formel (I) eine Wirkung gegen Schädlinge haben.

Überraschenderweise besitzen die erfindungsgemäßen 2-Cyano-2-alkoximino-acetamide der allgemeinen Formel (I) z.B. eine bessere fungizide Wirksamkeit als die aus dem Stand der Technik bekannte Verbindung Zink-ethylen-1,2-bis-dithiocarbamat, welches wirkungsmäßig eine naheliegende Verbindung ist.

Im einzelnen seien die bei den Herstellungsbeispielen aufgeführten Verbindungen genannt.

Verwendet man beispielsweise 3-(2-Cyano-2-oximino-acetamido)-5-methyl-isoxazol Natriumsalz und Dimethylsulfat als Ausgangsstoffe, so läßt sich der Reaktionsablauf des erfindungsgemäßen Verfahrens durch das folgende Formelschema darstellen:

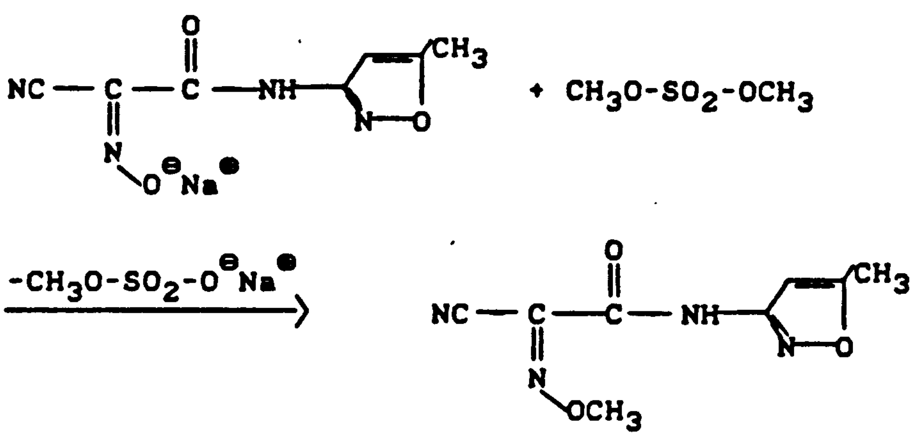


Die zur Durchführung des erfindungsgemäßen Verfahrens als Ausgangsstoffe benötigten 2-Cyano-2-oximino-acetamide sind durch die Formel (II) allgemein definiert. Bevorzugt sind Verbindungen der Formel (II), bei welchen $R^1$ für diejenigen Reste steht, die bereits im Zusammenhang mit der Beschreibung der erfindungsgemäßen Verbindungen der Formel (I) für diesen Substituenten genannt wurden und bei welchen M für Wasserstoff oder ein Natriumkation steht.

Die 2-Cyano-2-oximino-acetamide der Formel (II) sind noch nicht bekannt.

Man erhält sie in Analogie zu bekannten Verfahren (vgl. z.B. Ber. dtsch. chem. Ges. 42, 738 [1909] oder J. Pharm. Sci. 67, 860 [1978]), wenn man Cyanessigsäure oder aktivierte Derivate der Cyanessigsäure der Formel (IV),

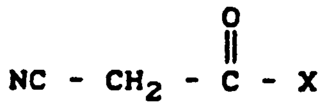

(IV)

in welcher

X für eine aktivierende Abgangsgruppe, wie beispielsweise Halogen, Methoxy, Ethoxy, Acetoxy oder Methansulfonyloxy, steht,

mit Aminen der Formel (V),

$H_2N - R^1$ (V)

in welcher

$R^1$ die oben angegebene Bedeutung hat,

gegebenenfalls in Gegenwart eines Verdünnungsmittels, wie beispielsweise Pyridin, Dimethylformamid oder Tetrahydrofuran und gegebenenfalls in Gegenwart eines Säurebindemittels, wie beispielsweise Triethylamin, bei Temperaturen zwischen -40 °C und +50 °C umsetzt. Man kann dabei aktivierte Derivate der Cyanessigsäure der Formel (IV), wie beispielsweise gemischte Anhydride mit Essigsäure oder Methansulfonsäure, auch in der Reaktionsmischung erst herstellen, indem man die Cyanessigsäure als solche einsetzt und z.B. Essigsäureanhydrid oder Methansulfonylchlorid in Gegenwart einer Base wie beispielsweise Pyridin zusetzt. Die so erhältlichen 2-Cyanacetamide der Formel (VI),

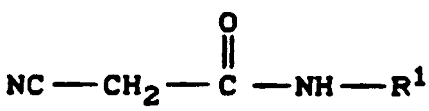

(VI)

in welcher

$R^1$ die oben angegebene Bedeutung hat,

werden in einer 2. Stufe mit einer Nitritverbindung der Formel (VII),

R-O-N = O (VII)

in welcher

R für ein Alkalimetallkation, insbesondere ein Natriumkation oder für einen Alkylrest, insbesondere ein Ethyl-, t-Butyl oder Isoamylrest steht,

gegebenenfalls in Gegenwart eines Verdünnungsmittels, wie beispielsweise Wasser, Methanol, Ethanol oder Tetrahydrofuran, gegebenenfalls in Gegenwart einer Katalysatorsäure, wie beispielsweise Chlorwasserstoffsäure oder Essigsäure oder alternativ in Gegenwart einer Base, wie beispielsweise Natriumamid, Natriummethylat oder Natriumethylat, bei Temperaturen zwischen -20 °C und +120 °C umgesetzt.

Cyanessigsäure und ihre aktivierten Derivate der Formel (IV), Amine der Formel (V) und Nitritverbindungen der Formel (VII) sind allgemein bekannte Verbindungen der organischen Chemie.

Die zur Durchführung des erfindungsgemäßen Verfahrens weiterhin als Ausgangsstoffe benötigten Alkylierungsmittel sind durch die Formel (III) allgemein definiert. In dieser Formel (III) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden.

A steht vorzugsweise für Halogen, insbesondere für Chlor, Brom oder Iod, oder für jeweils gegebenenfalls substituiertes Alkylsulfonyloxy, Alkoxysulfonyloxy oder Arylsulfonyloxy, wie beispielsweise Methansulfonyloxy, Methoxysulfonyloxy oder p-Toluolsulfonyloxy.

Die Alkylierungsmittel der Formel (III) sind allgemein bekannte Verbindungen der organischen Chemie.

Als Verdünnungsmittel zur Durchführung des erfindungsgemäßen Verfahrens kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder

Ethylenglykoldimethyl- oder diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid oder Ester, wie Essigsäureethylester; Sulfoxide, wie Dimethylsulfoxid, oder Alkohole wie Methanol, Ethanol oder Isopropanol.

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt : Tetrabutylammoniumiodid, Tetrabutylammoniumbromid, Tributyl-methylphosphoniumbromid, Trimethyl-$C_{13}/C_{15}$-alkylammoniumchlorid, Dibenzyldimethyl-ammonium-methylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des erfindungsgemäßen Verfahrens kommen alle üblicherweise verwendbaren anorganischen oder organischen Basen infrage. Vorzugsweise verwendet man Alkalimetallhydride, -hydroxide, -alkoholate, -amide, -carbonate oder -hydrogencarbonate, wie beispielsweise Natriumhydrid, Natriumamid, Natriumhydroxid, Natriumethylat, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -20 °C und 100 °C, vorzugsweise bei Temperaturen zwischen 0 °C und 60 °C.

Zur Durchführung des erfindungsgemäßen Verfahrens setzt man pro Mol an 2-Cyano-2-oximino-acetamid der Formel (II) im allgemeinen 1,0 bis 5,0 Mol, vorzugsweise 1,0 bis 3,0 Mol an Alkylierungsmittel der Formel (III) und 1,0 bis 5,0 Mol, vorzugsweise 1,0 bis 3,0 Mol an Säurebindemittel sowie gegebenenfalls 0,001 bis 1,0 Mol an Phasentransferkatalysator ein.

In einer bevorzugten Durchführungsform ist es auch möglich, die als Ausgangsstoffe verwendeten 2-Cyano-2-oximino-acetamide der Formel (II), wie bereits beschrieben, herzustellen und direkt aus dem Reaktionsgemisch heraus im "Eintopfverfahren" mit den Alkylierungsmitteln der Formel (III) weiter umzusetzen (vgl. Herstellungsbeispiele). Die Reaktionsdurchführung, Aufarbeitung und Isolierung der Reaktionsprodukte der Formel (I) erfolgt nach üblichen Methoden.

Die erfindungsgemäßen Wirkstoffe weisen eine starke Wirkung gegen Schädlinge auf und können zur Bekämpfung von unerwünschten Schädlingen praktisch eingesetzt werden. Die Wirkstoffe sind z.B. für den Gebrauch als Pflanzenschutzmittel geeignet.

So werden z.B. fungizide Mittel im Pflanzenschutz eingesetzt zur Bekämpfung von Plasmodiphoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;

Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Oomyceten, wie beispielsweise gegen den Erreger der Tomatenbraunfäule (Phytophthora infestans) oder gegen den Erreger des falschen Erbsenmehltaus (Peronospora pisi), oder zur Bekämpfung von Reiskrankheiten, wie beispielsweise gegen den Erreger der Reisfleckenkrankheit (Pyricularia oryzae) einsetzen. Besonders hervorzuheben ist, daß die erfindungsgemäßen Wirkstoffe nicht zur eine protektive Wirkung entfalten, sondern auch kurativ wirksam sind, also bei Anwendung nach der Kontamination der Pflanzen mit den Sporen des Pilzes.

Weiterhin ist auf die systemische Wirkung der Stoffe hinzuweisen. So gelingt es, Pflanzen gegen Pilzbefall zu schützen, indem man beispielsweise das Saatgut mit den Wirkstoffen beizt.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylaryl-polyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1:

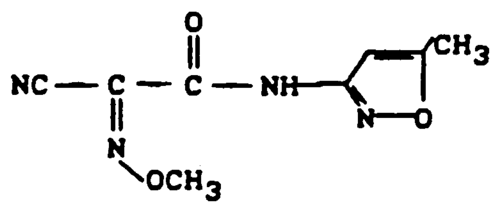

Zu 5 g (0,03 Mol) 3-Cyanacetamido-5-methyl-isoxazol und 3,5 g (0,03 Mol) Isoamylnitrit in 30 ml Ethanol gibt man bei 25 °C unter Rühren 2 g (0,03 Mol) Natriumethylat in 15 ml Ethanol und erwärmt für eine Minute auf 50 °C. Nach dem Abkühlen auf Raumtemperatur gibt man 3,8 g (0,03 Mol) Dimethylsulfat zu und läßt das Reaktionsgemisch eine Stunde stehen. Hierauf destilliert man das Lösungsmittel im Vakuum ab und chromatographiert den Rückstand mit Essigester als Laufmittel.

Man erhält 3,3 g (53 % der Theorie) 3-(2-Cyano-2-methoximino-acetamido)-5-methyl-isoxazol vom Schmelzpunkt 197 ° - 199 °C.

Herstellung der Ausgangsverbindung

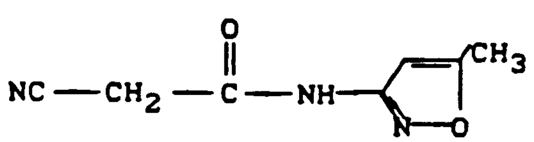

Zu 8,5 g (0,1 Mol) Cyanessigsäure und 9,8 g (0,1 Mol) 3-Amino-5-methyl-isoxazol in 50 ml Pyridin gibt man bei Raumtemperatur 10,2 g (0,1 Mol) Acetanhydrid in 50 ml Pyridin. Sofort anschließend wird das Pyridin im Vakuum entfernt, der Rückstand in Toluol aufgenommen, eingeengt, ein zweites Mal in Toluol aufgenommen, wieder eingeengt, mit 100 ml Wasser behandelt, abgesaugt, mit Wasser gewaschen und getrocknet.

Man erhält 15,3 g (93 % der Theorie) an 3-Cyanacetamido-5-methyl-isoxazol vom Schmelzpunkt 225 ° - 227 °C (Zers.).

Beispiel 2:

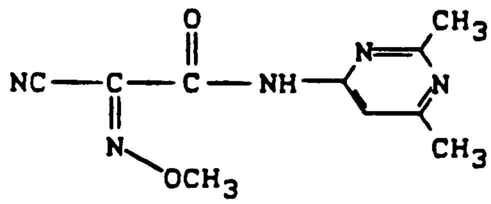

Zu einer Lösung von 5,7 g (0,03 Mol) 2,6-Dimethyl-4-cyanacetamido-pyrimidin in 30 ml Ethanol gibt man 3,5 g (0,03 Mol) Isoamylnitrit und danach 2 g (0,03 Mol) Natriumethylat in 15 ml Ethanol und läßt die Mischung bei 25 °C 30 Minuten stehen. Hierauf gibt man 3,8 g (0,03 Mol) Dimethylsulfat zu und läßt die Mischung eine weitere Stunde bei Raumtemperatur stehen. Nach dem Abdestillieren des Lösungsmittels im Vakuum chromatographiert man den Rückstand mit Essigester als Laufmittel.

Man erhält 2,3 g (33 % der Theorie) 2,6-Dimethyl-4-(2-cyano-2-methoximino-acetamido)-pyrimidin vom Schmelzpunkt 128 °C.

Herstellung der Ausgangsverbindung

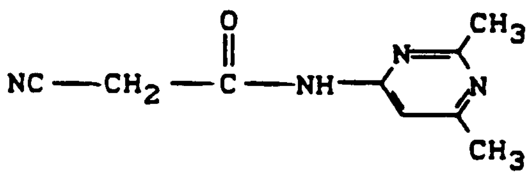

6,9 g (0,08 Mol) Cyanessigsäure und 10 g (0,08 Mol) 4-Amino-2,6-dimethylpyrimidin werden in 80 ml Pyridin gelöst und mit 8,3 g (0,08 Mol) Essigsäureanhydrid verzetzt. Anschließend entfernt man das Pyridin im Vakuum, nimmt den Rückstand in Toluol auf, engt ein, nimmt ein zweites Mal in Toluol auf und engt wieder ein. Den Rückstand chromatographiert man mit Essigsäureethylester als Laufmittel.

Man erhält 7,8 g (50,5 % der Theorie) 2,6-Dimethyl-4-cyanacetamido-pyrimidinvom Schmelzpunkt 178 °C.

Beispiel 3:

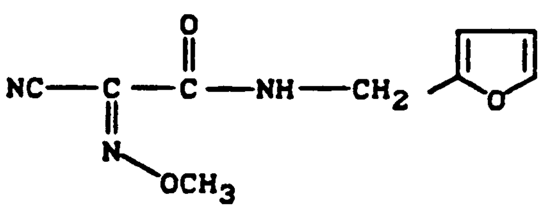

Zu 8,2 g (0,05 Mol) Cyanessigsäure-2-furfurylamid und 5,9 g (0,05 Mol) Isoamylnitrit in 25 ml Ethanol gibt man 3,4 g (0,05 Mol) Natriumethylat in 25 ml Ethanol und läßt die Reaktionsmischung eine Stunde bei Raumtemperatur stehen. Hierauf gibt man 6,3 g (0,05 Mol) Dimethylsulfat zu und läßt das Gemisch für weitere 12 Stunden bei Raumtemperatur stehen. Nach dem Abdestillieren des Lösungsmittels chromatographiert man den Rückstand mit Essigsäureethylester als Laufmittel.

Man erhält 8,7 g (87 % der Theorie) 2-Methoximino-cyanessigsäure-2-furfurylamid als Öl.

$^1H$-NMR ($CDCl_3$) : $\delta$ = 4,25 (s); 4,52 (d); 6,31; 6,35; 6,97 (breit); 7,38 ppm

Herstellung der Ausgangsverbindung

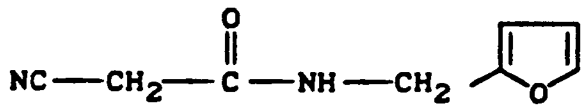

Zu einer Lösung von 4,9 g (0,05 Mol) 2-Furfurylamin und 4,3 g (0,05 Mol) Cyanessigsäure in 25 ml Pyridin gibt man 5,8 g (0,05 Mol) Methansulfonsäurechlorid in 25 ml Pyridin, destilliert dann das Pyridin im Vakuum ab, versetzt den Rückstand mit 25 ml Toluol, engt im Vakuum ein, versetzt ein zweites Mal mit 25 ml Toluol und engt wiederum im Vakuum ein, behandelt den Rückstand mit 50 ml Wasser, saugt ab, wäscht zweimal mit je 20 ml Wasser nach und trocknet.

Man erhält 4,5 g (55 % der Theorie) an Cyanessigsäure-2-furfurylamid vom Schmelzpunkt 93 ° - 95 ° C.

Beispiel 4:

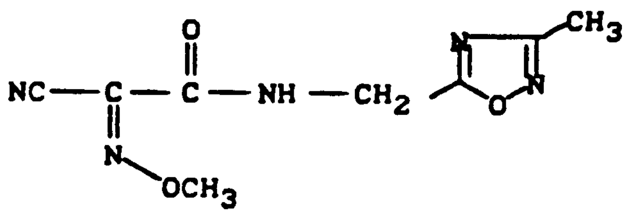

Zu 16 g (0,089 Mol) 5-Cyanacetamidomethyl-3-methyl-1,2,4-oxadiazol in 80 ml absolutem Ethanol tropft man unter Rühren bei Raumtemperatur zunächst 10,42 g (0,089 Mol) Isopentylnitrit und anschließend 6,0 g (0,089 Mol) Natriumethylat in 50 ml absolutem Ethanol. Das Gemisch wird für eine Stunde bei Raumtemperatur nachgerührt, dann tropfenweise mit 11,22 g (0,089 Mol) Dimethylsulfat versetzt und eine weitere Stunde bei Raumtemperatur nachgerührt. Zur Aufarbeitung entfernt man das Lösungsmittel im Vakuum, nimmt den Rückstand in Dichlormethan auf, wäscht mehrmals mit Wasser, trocknet über Natriumsulfat, entfernt das Lösungsmittel im Vakuum und reinigt den Rückstand chromatographisch an Kieselgel (Laufmittel : Dichlormethan/Ether 10 : 1).

Man erhält 8,2 g (41 % der Theorie) an 5-(2-Cyano-2-methoximino-acetamidomethyl)-3-methyl-1,2,4-oxadiazol vom Schmelzpunkt 69 ° C.

Herstellung der Ausgangsverbindung

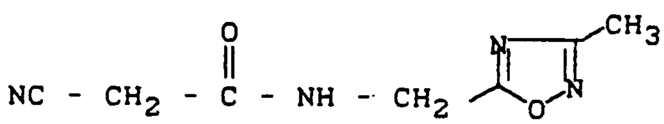

10,0 g (0,067 Mol) 5-Aminomethyl-3-methyl-1,2,4-oxadiazol werden in 300 ml absolutem Tetrahydrofuran vorgelegt und mit 6,8 g (0,067 Mol) Triethylamin versetzt. Man läßt ca. 15 Minuten bei Raumtemperatur rühren und setzt anschließend unter Eisbadkühlung 3,47 g (0,00335 Mol) Cyanacetylchlorid zu, läßt ca. 5 Minuten rühren, setzt 3,4 g (0,00336 Mol) Triethylamin zu und dann anschließend im iterativen Verfahren abwechselnd insgesamt 6,93 g (0,067 Mol) Cyanacetylchlorid und 6,8 g (0,067 Mol) Triethylamin zu.
Zur Aufarbeitung wird vom ausgefallenen Triethylaminhydrochlorid abfiltriert, das Lösungsmittel im Vakuum abgedampft und der braune, ölige Rückstand roh weiter umgesetzt.

Man erhält 12,0 g (99,5% der Theorie) an 5-Cyanacetamidomethyl-3-methyl-1,2,4-oxadiazol.

In entsprechender Weise und gemäß den allgemeinen Angaben zur Herstellung erhält man die

folgenden 2-Cyano-2-alkoximino-acetamide der allgemeinen Formel (I):

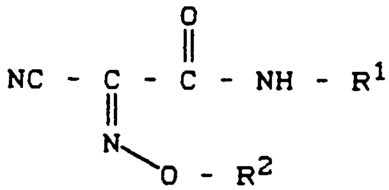 (I)

| Bsp. Nr. | $R^1$ | $R^2$ | physikalische Eigenschaften |
|---|---|---|---|
| 5 | 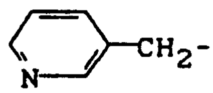 | $-CH_3$ | Fp. 136°-137° C |
| 6 | 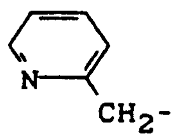 | $-CH_3$ | Fp. 85°-88° C |
| 7 | 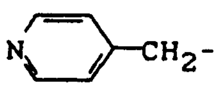 | $-CH_3$ | Fp. 135°-139° C |
| 8 | 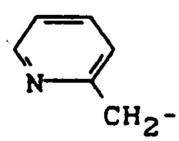 | 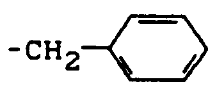 | Fp. 90°-92° C |
| 9 | 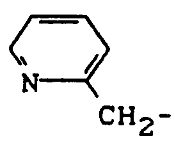 | 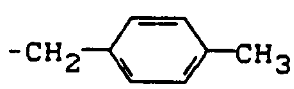 | Fp. 94°-95° C |

| Bsp. Nr. | $R^1$ | $R^2$ | physikalische Eigenschaften |
|---|---|---|---|
| 10 | 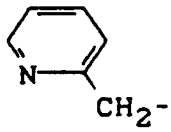 | $-(CH_2)_{11}-CH_3$ | Fp. 50° C |
| 11 | 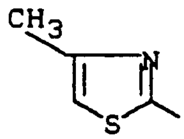 | $-CH_3$ | Fp. 53° -54° C |
| 12 | 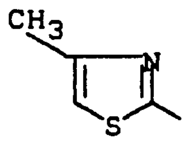 | $-CH_2$- 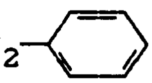 | Fp. 98° C |
| 13 | 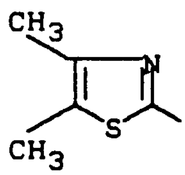 | $-CH_3$ | Fp. 117° C |
| 14 | 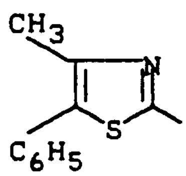 | $-CH_3$ | Fp. 180° C |
| 15 | 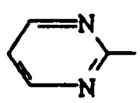 | $-CH_3$ | Fp. 138° C |
| 16 | 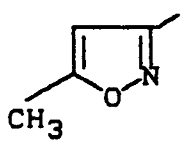 | $-CH_2-CN$ | Fp. 182° C |
| 17 | 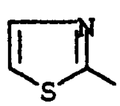 | $-CH_3$ | Fp. 220° C(Zers.) |

| Bsp. Nr. | $R^1$ | $R^2$ | physikalische Eigenschaften |
|---|---|---|---|
| 18 | 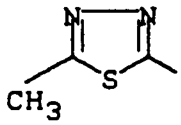 | $-CH_3$ | Fp. 210° C |
| 19 | 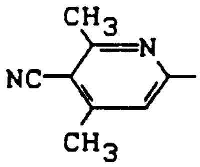 | $-CH_3$ | Fp. 196° C |
| 20 | 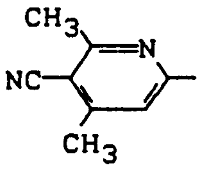 | $-CH_2-CN$ | Fp. 218° C |
| 21 | 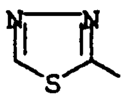 | $-CH_2-CN$ | Fp. 162° C |
| 22 | 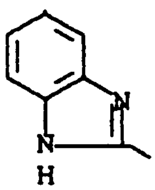 | $-CH_3$ | Fp. 172° C |
| 23 | 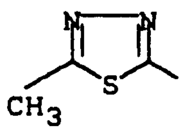 | $-CH_2-CN$ | Fp. 228° C(Zers.) |
| 24 | 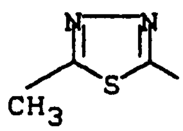 | $-CH_2$ 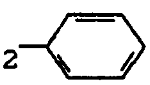 | Fp. 228° C(Zers.) |
| 25 | 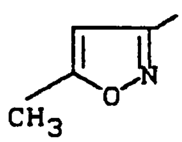 | $-CH_2$ 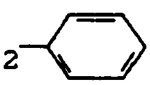 | Fp. 164° C |

| Bsp. Nr. | $R^1$ | $R^2$ | physikalische Eigenschaften |
|---|---|---|---|
| 26 | 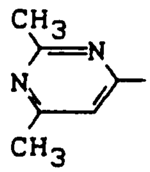 | $-CH_3$ | Fp. 120° -125° C |
| 27 | 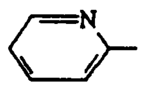 | $-CH_3$ | Fp. 105° -110° C |
| 28 | 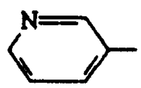 | $-CH_3$ | Fp. 142° -143° C |
| 29 | 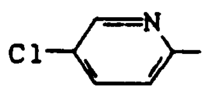 | $-CH_3$ | Fp. 139° -142° C |
| 30 | 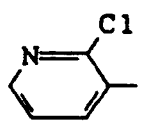 | $-CH_3$ | Fp. 113° -115° C |
| 31 | 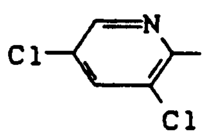 | $-CH_3$ | Fp. 142° -143° C |
| 32 | 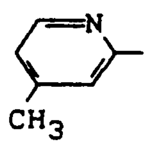 | $-CH_3$ | Fp. 124° -130° C |
| 33 | 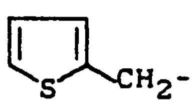 | $-CH_3$ | NMR*) : 4,23<br>4,7 |
| 34 | 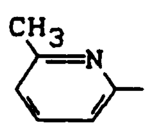 | $-CH_3$ | Fp. 115° -117° C |

| Bsp. Nr. | $R^1$ | $R^2$ | physikalische Eigenschaften |
|---|---|---|---|
| 35 | 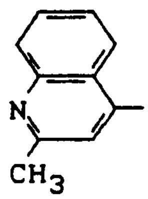 | $-CH_3$ | Fp. 187°-191° C |
| 36 | 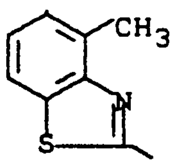 | $-CH_3$ | Fp. 215°-218° C |
| 37 | 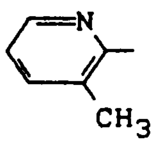 | $-CH_3$ | NMR*): 2.18<br>4.27 |
| 38 | 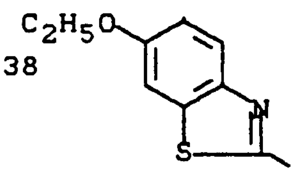 | $-CH_3$ | NMR*): 1.38<br>4.28 |
| 39 | 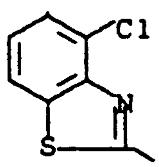 | $-CH_3$ | NMR*): 4.28 |
| 40 | 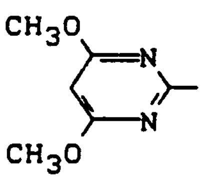 | $-CH_3$ | Fp. 140°-142° C |

| Bsp. Nr. | $R^1$ | $R^2$ | physikalische Eigenschaften |
|---|---|---|---|
| 41 | 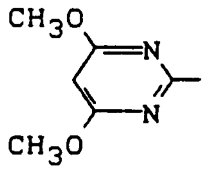 | $-CH_2-$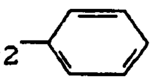 | Fp. 112° -113° C |
| 42 | 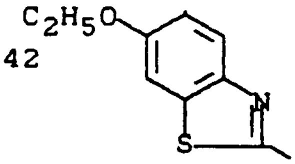 | $-CH_2-$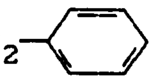 | Fp. 139° -140° C |
| 43 | 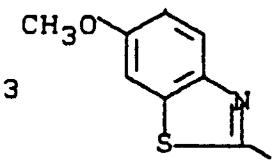 | $-CH_3$ | NMR*) : 3.83<br>4.28 |
| 44 | 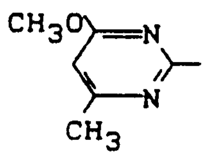 | $-CH_3$ | Fp. 163° -165° C |
| 45 | 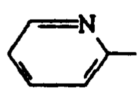 | $-CH_2-$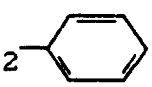 | Fp. 84° C-85° C |
| 46 | 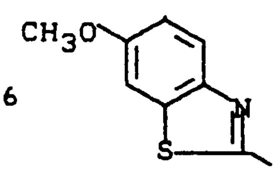 | $-CH_2-$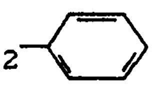 | NMR*) : 3.83<br>5.5 |
| 47 | 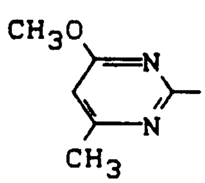 | $-CH_2-$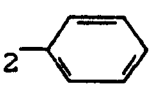 | NMR*) : 3.95<br>5.5 |

| Bsp. Nr. | $R^1$ | $R^2$ | physikalische Eigenschaften |
|---|---|---|---|
| 48 | 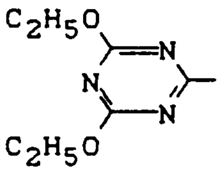 | $-CH_3$ | NMR*) : 4.32<br>4.52 |
| 49 | 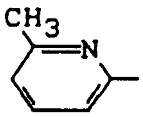 | $-CH_2$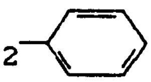 | Fp. 121° -123° C |
| 50 | 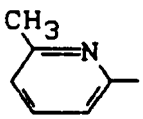 | $-CH_2-CN$ | Fp. 125 °C |
| 51 | 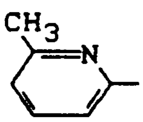 | $-C_2H_5$ | NMR*) : 2.45<br>4.57 |
| 52 | 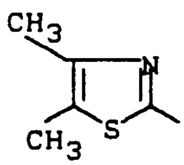 | $-C_2H_5$ | Fp. 140° -141° C |
| 53 | 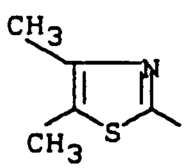 | $-(CH_2)_{11}-CH_3$ | NMR*) : 0.88<br>3.43 |
| 54 | $HO-(CH_2)_3-$ | $-C_2H_5$ | NMR*) : 1.42<br>4.48 |
| 55 | $HO-(CH_2)_3-$ | $-CH_2$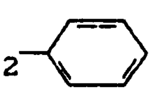 | NMR*) : 3.69<br>5.38 |
| 56 | $HO-(CH_2)_3-$ | $-(CH_2)_2-CH_3$ | NMR*) : 3.75<br>4.41 |

| Bsp. Nr. | $R^1$ | $R^2$ | physikalische Eigenschaften |
|---|---|---|---|
| 57 | $HO-(CH_2)_3-$ | $-(CH_2)_{11}-CH_3$ | NMR*) : 3.72<br>4.42 |
| 58 | $HO-(CH_2)_3-$ | O<br>‖<br>$-(CH_2)_2-O-C-CH_3$ | NMR*) : 2.09<br>3.73 |
| 59 | $HO-(CH_2)_3-$ | $CH_3$ O<br>\| ‖<br>$-CH - C-CH_3$ | NMR*) : 3.77<br>4.9 |
| 60 | $HO-(CH_2)_3-$ | O<br>‖<br>$-CH_2-C-O-C_2H_5$ | NMR*) : 3.72<br>4.95 |
| 61 | $HO-(CH_2)_3-$ | $-CH_2-CH=CH-CH_3$ | NMR*) : 3.72<br>4.85 |
| 62 | $HO-(CH_2)_3-$ | $-CH_2-CH=CH_2$ | NMR*) : 3.74<br>4.9 |
| 63 | $HO-(CH_2)_3-$ | $-CH_2-CH=C\langle^{Cl}_{CH_3}$ | NMR*) : 3.73<br>2.2 |
| 64 | $HO-(CH_2)_3-$ | $-CH_2-C\equiv CH$ | NMR*) : 2.71<br>3.72<br>5.0 |
| 65 | $HO-(CH_2)_3-$ | $-(CH_2)_2-CH=CH_2$ | NMR*) : 3.73<br>4.48 |

| Bsp. Nr. | $R^1$ | $R^2$ | physikalische Eigenschaften |
|---|---|---|---|
| 66 | $HO-(CH_2)_3-$ | 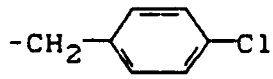 | Fp. 90° C |
| 67 | $HO-(CH_2)_3-$ | 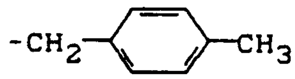 | NMR*) : 3.7<br>5.34 |
| 68 | $HO-(CH_2)_3-$ | 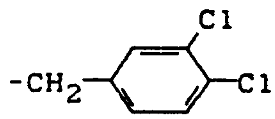 | Fp. 94°-95° C |
| 69 | $HO-(CH_2)_2-$ | $-C_2H_5$ | NMR*) : 3.78<br>4.5 |
| 70 | $HO-(CH_2)_2-$ | $-(CH_2)_2-CH_3$ | NMR*) : 3.78<br>4.41 |
| 71 | $HO-(CH_2)_2-$ | $-(CH_2)_{11}-CH_3$ | NMR*) : 3.8<br>4.43 |
| 72 | $HO-(CH_2)_2-$ | 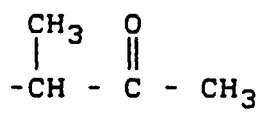 | NMR*) : 3.82<br>4.92 |
| 73 | $HO-(CH_2)_2-$ | 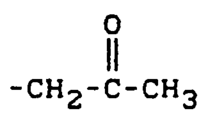 | NMR*) : 3.8 |
| 74 | $HO-(CH_2)_2-$ | 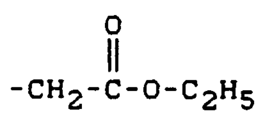 | NMR*) : 3.75<br>4.92 |
| 75 | $HO-(CH_2)_2-$ | 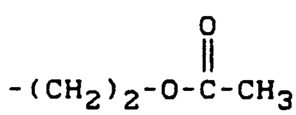 | NMR*) : 2.12<br>3.78 |

| Bsp. Nr. | $R^1$ | $R^2$ | physikalische Eigenschaften |
|---|---|---|---|
| 76 | $HO-(CH_2)_2-$ | $-CH_2-CH=CH_2$ | NMR*) : 3.77<br>4.91 |
| 77 | $HO-(CH_2)_2-$ | $-CH_2-CH=CH-CH_3$ | NMR*) : 1.75<br>3.8 |
| 78 | $HO-(CH_2)_2-$ | $-CH_2-CH=C(Cl)CH_3$ | NMR*) : 2.21<br>3.78 |
| 79 | $HO-(CH_2)_2-$ | $-CH_2-C\equiv CH$ | NMR*) : 2.65<br>3.72 |
| 80 | $HO-(CH_2)_2-$ | $-(CH_2)_2-CH=CH_2$ | NMR*) : 3.79<br>4.5 |
| 81 | $HO-(CH_2)_2-$ | $-CH_2-$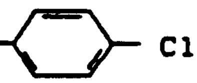 | Fp. 112° -114° C |
| 82 | $HO-(CH_2)_2-$ | $-CH_2-$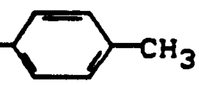 | Fp. 100° C |
| 83 | $HO-(CH_2)_2-$ | $-CH_2-$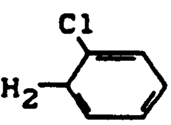 | Fp. 98° -103° C |
| 84 | $HO-(CH_2)_2-$ | $-CH_2-$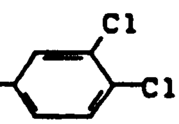 | Fp. 120° -121° C |
| 85 | $HO-(CH_2)_2-$ | $-CH_2-$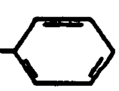 | NMR*) : 3.77<br>5.42 |

| Bsp. Nr. | $R^1$ | $R^2$ | physikalische Eigenschaften |
|---|---|---|---|
| 86 | $CH_3-CH(OH)-CH_2-$ | $-C_2H_5$ | NMR*) : 1.22<br>4.51 |
| 87 | $CH_3-CH(OH)-CH_2-$ | $-(CH_2)_2-CH_3$ | NMR*) : 1.22<br>4.42 |
| 88 | $CH_3-CH(OH)-CH_2-$ | $-CH_2-C(=O)-OC_2H_5$ | NMR*) : 1.22<br>4.93 |
| 89 | $CH_3-CH(OH)-CH_2-$ | $-CH_2-CH_2-O-C(=O)-CH_3$ | NMR*) : 1.22<br>2.11 |
| 90 | $CH_3-CH(OH)-CH_2-$ | $-CH_2-CH=CH_2$ | NMR*) : 1.22<br>4.9 |
| 91 | $CH_3-CH(OH)-CH_2-$ | $-CH_2-CH=CH-CH_3$ | NMR*) : 1.22<br>2.21 |
| 92 | $CH_3-CH(OH)-CH_2-$ | $-CH_2-CH=C(Cl)CH_3$ | NMR*) ; 1.22<br>2.2 |
| 93 | $CH_3-CH(OH)-CH_2-$ | $-CH_2-C\equiv CH$ | NMR*) : 1.22<br>5.0 |
| 94 | $HO-(CH_2)_2-$ | $CH_3$ | Fp. 66° -69° C |

| Bsp. Nr. | $R^1$ | $R^2$ | physikalische Eigenschaften |
|---|---|---|---|
| 95 | OH<br>\|<br>$CH_3-CH-CH_2-$ | $-(CH_2)_2-CH=CH_2$ | NMR*): 1.22<br>4.5 |
| 96 | OH<br>\|<br>$CH_3-CH-CH_2-$ | 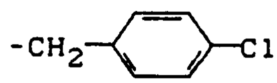 | Fp. 93° C |
| 97 | OH<br>\|<br>$CH_3-CH-CH_2-$ | 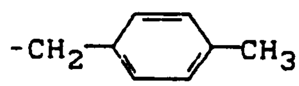 | Fp. 92° C |
| 98 | OH<br>\|<br>$CH_3-CH-CH_2-$ | 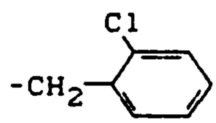 | NMR*): 1.22<br>5.57 |
| 99 | OH<br>\|<br>$CH_3-CH-CH_2-$ | 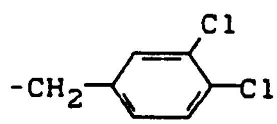 | Fp. 87° C |
| 100 | OH<br>\|<br>$CH_3-CH-CH_2-$ | 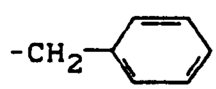 | Fp. 78° C-80° C |
| 101 | OH<br>\|<br>$CH_3-CH-(CH_2)_2-$ | $-C_2H_5$ | NMR*): 1.22<br>4.48 |
| 102 | 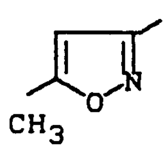 | $-C_2H_5$ | Fp. 157°-158° C |
| 103 | 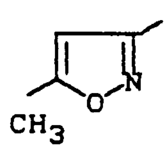 | $-(CH_2)_2-CH_3$ | Fp. 142°-146° C |

| Bsp. Nr. | $R^1$ | $R^2$ | physikalische Eigenschaften |
|---|---|---|---|
| 104 | 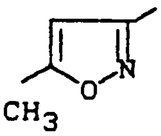 | $-(CH_2)_{11}-CH_3$ | Fp. 112° -114° C |
| 105 | 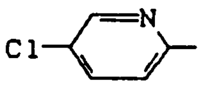 | $-C_2H_5$ | Fp. 78° -80° C |
| 106 | 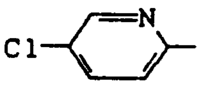 | $-(CH_2)_2-CH_3$ | NMR*) : 1.87<br>4.47 |
| 107 | 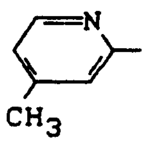 | $-CH_2$ 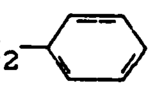 | Fp. 98° -100° C |
| 108 | 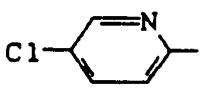 | 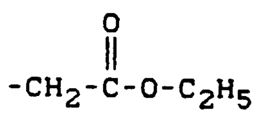 | Fp. 106° -108° C |
| 109 | 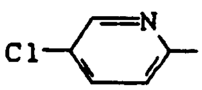 | $-CH_2$ 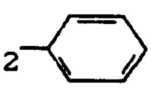 | Fp. 114° -116° C |
| 110 | 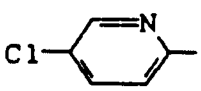 | $-(CH_2)_{11}-CH_3$ | Fp. 73° -74° C |
| 111 | 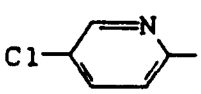 | $-CH_2$ 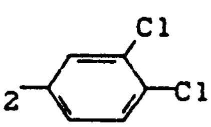 | Fp. 154° -156° C |
| 112 | 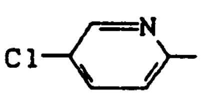 | $-CH_2-CH=CH_2$ | Fp. 75° -76° C |

| Bsp. Nr. | $R^1$ | $R^2$ | physikalische Eigenschaften |
|---|---|---|---|
| 113 | 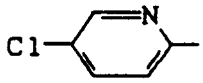 | $-CH_2-CH=CH-CH_3$ | Fp. 59° -62° C |
| 114 | 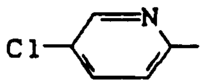 | 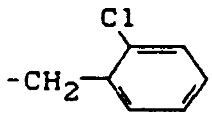 | Fp. 139° -140° C |
| 115 | 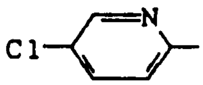 | 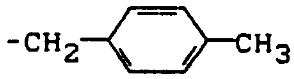 | Fp. 117-118° C |
| 116 | 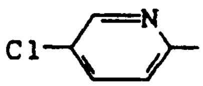 | 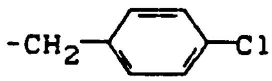 | Fp. 146° -147° C |
| 117 | 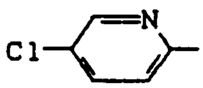 | 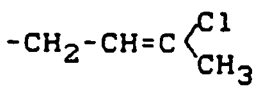 | Fp. 79° -81° C |
| 118 | 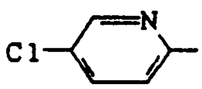 | 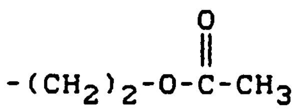 | NMR*) : 2.12 |
| 119 | 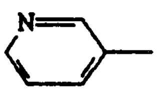 | 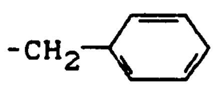 | Fp. 110° -111° C |
| 120 | 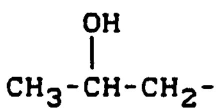 | $CH_3$ | Kp. 125° C/0.7mm |
| 121 | 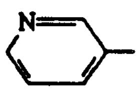 | $-CH_2-CN$ | Fp. 116° -118° C |
| 122 | 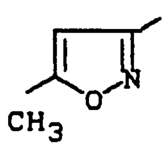 | 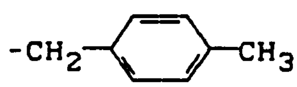 | Fp. 177° -178° C |

| Bsp. Nr. | $R^1$ | $R^2$ | physikalische Eigenschaften |
|---|---|---|---|
| 123 | 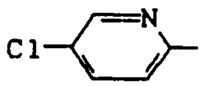 | 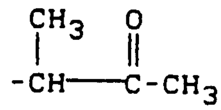 | Fp. 102° -104° C |
| 124 | 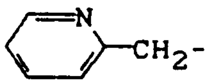 | $-CH_2-CN$ | Fp. 107° -109° C |
| 125 | 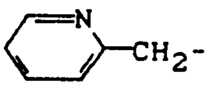 | $-C_2H_5$ | NMR*) :1.38 |
| 126 | 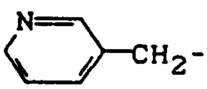 | 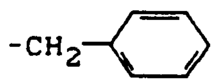 | Fp. 95° C-96° C |
| 127 | 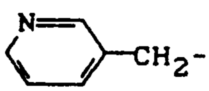 | $-(CH_2)_2-CH_3$ | Fp. 120° -122° C |
| 128 | 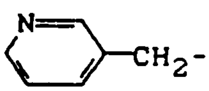 | $-(CH_2)_{11}-CH_3$ | Fp. 68° -72° C |
| 129 | 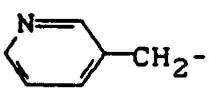 | 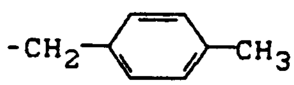 | Fp. 53° -55° C |
| 130 | 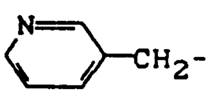 | 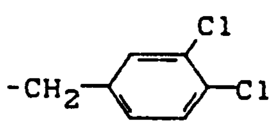 | Fp. 100° -103° C |
| 131 | 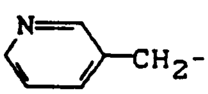 | 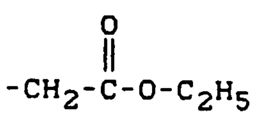 | Fp. 147° -149° C |
| 132 | 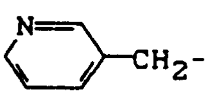 | 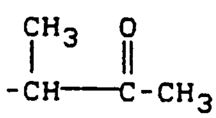 | Fp. 100° -101° C |

| Bsp. Nr. | $R^1$ | $R^2$ | physikalische Eigenschaften |
|---|---|---|---|
| 133 | 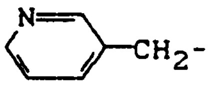 | 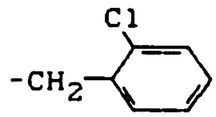 | Fp. 119° -122° C |
| 134 | 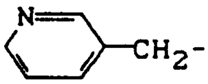 | 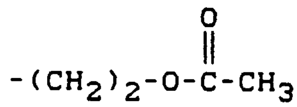 | Fp. 73° -77° C |
| 135 | 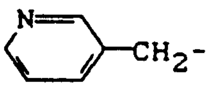 | 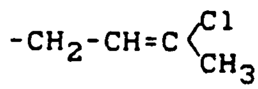 | Fp. 98° -100° C |
| 136 | 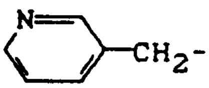 | $-CH_2-CH=CH_2$ | Fp. 74° -76° C |
| 137 | 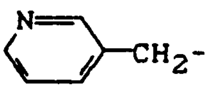 | 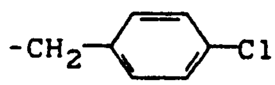 | Fp. 128° C |
| 138 | 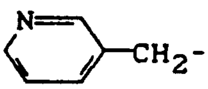 | $-CH_2-CH=CH-CH_3$ | Fp. 71° C |
| 139 | 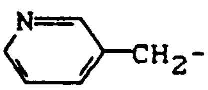 | $-(CH_2)_2-CH=CH_2$ | NMR*) : 4.42<br>4.48 |
| 140 | 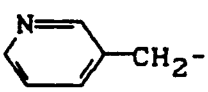 | 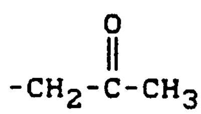 | NMR*) : 4.48<br>6.02 |
| 141 | 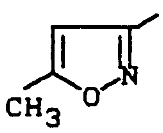 | 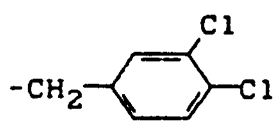 | NMR*) : 2.52<br>5.55 |

| Bsp. Nr. | $R^1$ | $R^2$ | physikalische Eigenschaften |
|---|---|---|---|
| 142 | 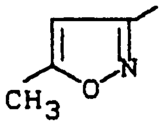 | $-CH_2$ 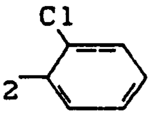 | Fp. 168° - 170° C |
| 143 | 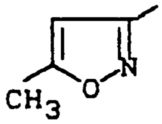 | $-CH_2$ 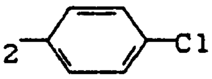 | Fp. 165° - 167° C |
| 144 | 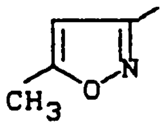 | $-CH_2-CH=CH_2$ | Fp. 122° - 126° C |
| 145 | 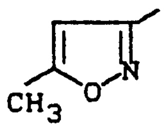 | $-CH_2-\overset{O}{\overset{\|}{C}}-O-C_2H_5$ | Fp. 175° - 176° C |
| 146 | 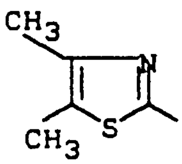 | $-CH_2$ 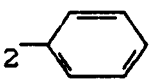 | Fp. 156° - 158° C |
| 147 | 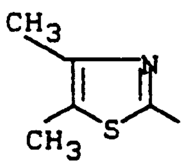 | $-(CH_2)_2-CH_3$ | Fp. 91° - 96° C |
| 148 | 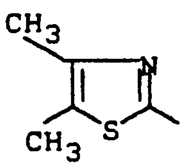 | $-CH_2-CN$ | Fp. 178° - 180° C |

| Bsp. Nr. | $R^1$ | $R^2$ | physikalische Eigenschaften |
|---|---|---|---|
| 149 | 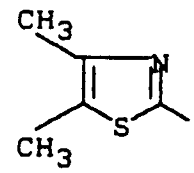 | 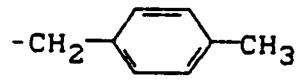 | Fp. 158°-159° C |
| 150 | 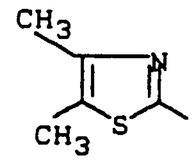 | 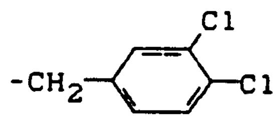 | NMR*) : 5.32 |
| 151 | 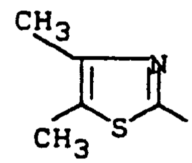 | 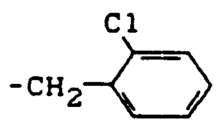 | Fp. 158° C |
| 152 | 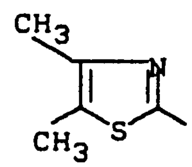 | $-CH_2-CH=CH_2$ | Fp. 111°-112° C |
| 153 | 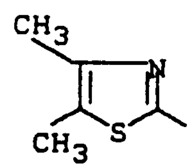 | $-CH_2-CH=CH-CH_3$ | Fp. 118° C |
| 154 | 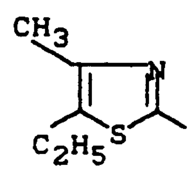 | 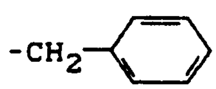 | Fp. 123° C |
| 155 | 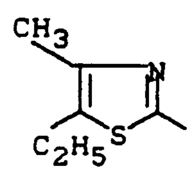 | $-CH_3$ | Fp. 151°-5° C |

| Bsp. Nr. | $R^1$ | $R^2$ | physikalische Eigenschaften |
|---|---|---|---|
| 156 | 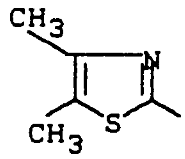 | $-(CH_2)_2-CH=CH_2$ | Fp. 104° -105° C |
| 157 | 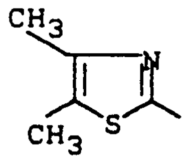 | 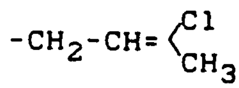 | NMR*) : 5.48-6.00 |
| 158 | 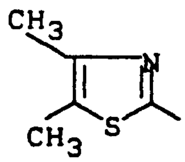 | 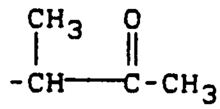 | Fp. 124° -126° C |
| 159 | 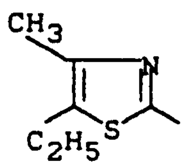 | $-C_2H_5$ | Fp. 139° -140° C |
| 160 | 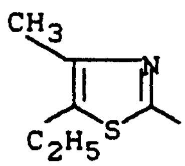 | $-(CH_2)_2-CH_3$ | Fp. 99° -100° C |
| 161 | 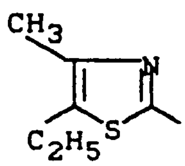 | $-(CH_2)_{11}-CH_3$ | Fp. 50° C |
| 162 | 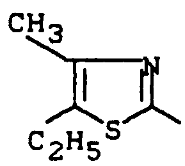 | $-CH_2-CN$ | NMR*) : 2.24 5.14 |

| Bsp. Nr. | $R^1$ | $R^2$ | physikalische Eigenschaften |
|---|---|---|---|
| 163 | 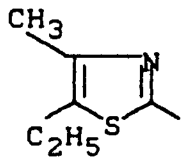 | 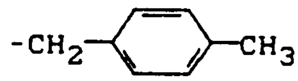 | Fp. 119° C |
| 164 | 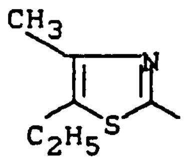 | 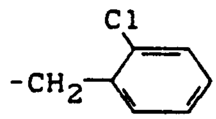 | Fp. 113° -114° C |
| 165 | 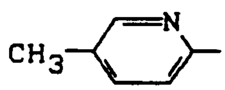 | $CH_3$ | Fp. 112° -114° C |
| 166 | 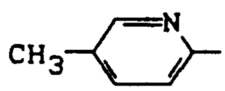 | 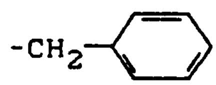 | Fp. 97° -99° C |
| 167 | 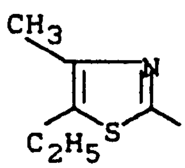 | 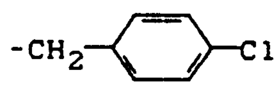 | Fp. 140° -143° C |
| 168 | 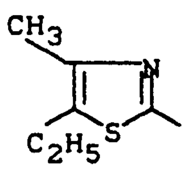 | 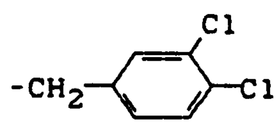 | Fp. 153° -154° C |
| 169 | 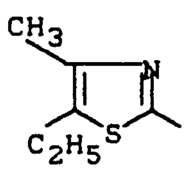 | $-CH_2-CH=CH_2$ | Fp. 83° -84° C |

| Bsp. Nr. | $R^1$ | $R^2$ | physikalische Eigenschaften |
|---|---|---|---|
| 170 | 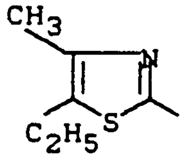 | $-CH_2-CH=CH-CH_3$ | Fp. 112°-114° C |
| 171 | 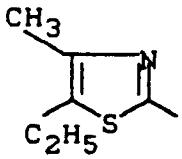 | $-(CH_2)_2-CH=CH_2$ | Fp. 102°-105° C |
| 172 | 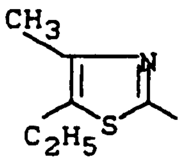 | 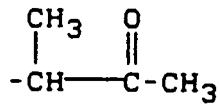 | Fp. 119°-122° C |
| 173 | 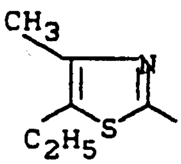 | 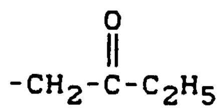 | Fp. 82°-83° C |
| 174 | 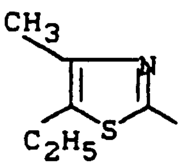 | 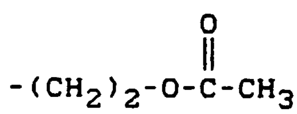 | Fp. 102°-103° C |
| 175 | 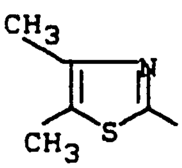 | 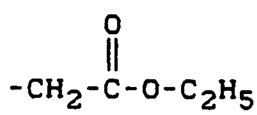 | Fp. 149°-151° C |
| 176 | 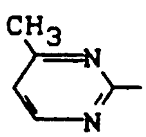 | 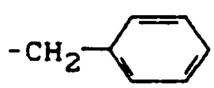 | Fp. 91°-92° C |

| Bsp. Nr. | $R^1$ | $R^2$ | physikalische Eigenschaften |
|---|---|---|---|
| 177 | 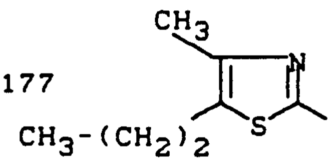 | $-CH_3$ | Fp. 123° -124° C |
| 178 | 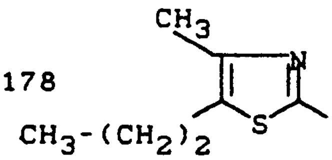 | $-CH_2-$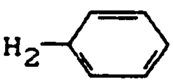 | Fp. 82° -85° C |
| 179 | 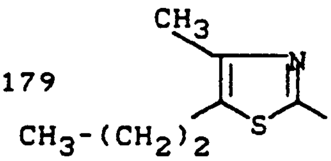 | $-CH_2-CN$ | Fp. 122° -124° C |
| 180 | 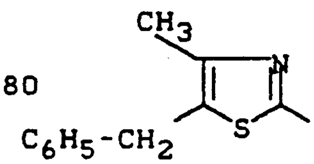 | $-CH_3$ | Fp. 117° -118° C |
| 181 | 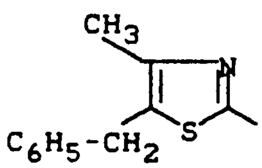 | $-CH_2-$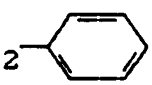 | Fp. 140° -141° C |
| 182 | 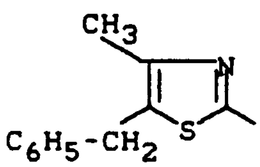 | $-CH_2-CN$ | Fp. 162° -163° C |
| 183 | 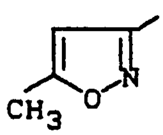 | $-CH_2-CH=CH-CH_3$ | Fp. 143° C |

| Bsp. Nr. | $R^1$ | $R^2$ | physikalische Eigenschaften |
|---|---|---|---|
| 184 | 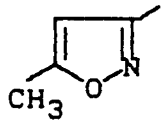 | $-(CH_2)_2-CH=CH_2$ | Fp. 152°-154° C |
| 185 | 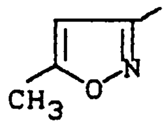 | $-CH_2-CH=C(Cl)CH_3$ | Fp. 128°-130° C |
| 186 | 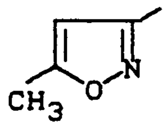 | $-(CH_2)_2-O-C(=O)-CH_3$ | Fp. 140°-143° C |
| 187 | 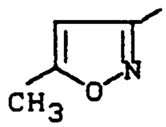 | $-CH_2-C\equiv CH$ | Fp. 144°-146° C |
| 188 | $CH_3-CH(OH)-(CH_2)_2-$ | $-(CH_2)_2-CH_3$ | NMR*) : 1.22<br>4.4 |
| 189 | $CH_3-CH(OH)-(CH_2)_2-$ | $-(CH_2)_{11}-CH_3$ | NMR*) : 0.9<br>4.4 |
| 190 | $CH_3-CH(OH)-(CH_2)_2-$ | $-CH_2-C(=O)-O-C_2H_5$ | NMR*) : 1.23<br>4.92 |
| 191 | $CH_3-CH(OH)-(CH_2)_2-$ | $-(CH_2)_2-O-C(=O)-CH_3$ | NMR*) : 1.25<br>2.12 |

| Bsp. Nr. | $R^1$ | $R^2$ | physikalische Eigenschaften |
|---|---|---|---|
| 192 | 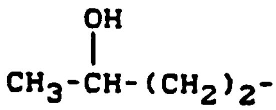 | $-CH_2-CH=CH_2$ | NMR*) : 1.23<br>4.9 |
| 193 | 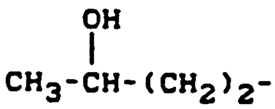 | $-CH_2-CH=CH-CH_3$ | NMR*) : 1.25<br>1.8 |
| 194 | 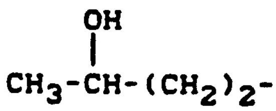 | 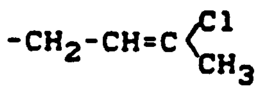 | NMR*) : 1.23<br>2.2 |
| 195 | 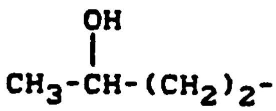 | $-CH_2-C\equiv CH$ | NMR*) : 1.23<br>4.98 |
| 196 | 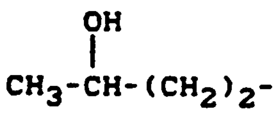 | $-CH_2-CH_2-CH=CH_2$ | NMR*) : 1.23<br>4.48 |
| 197 | 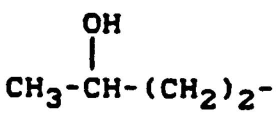 | 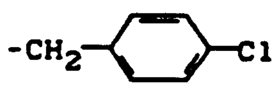 | NMR*) : 1.22<br>5.35 |
| 198 | 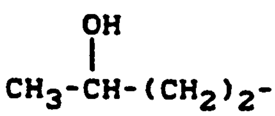 | 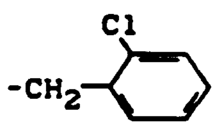 | NMR*) : 1.22<br>5.52 |
| 199 | 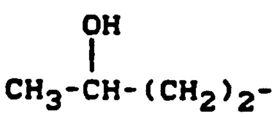 | 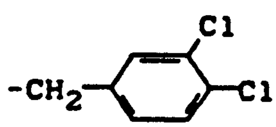 | NMR*) : 1.23<br>5.32 |

| Bsp. Nr. | $R^1$ | $R^2$ | physikalische Eigenschaften |
|---|---|---|---|
| 200 | 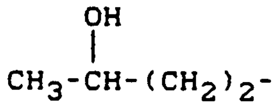 | 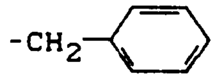 | NMR*) : 1.23<br>5.41 |
| 201 | 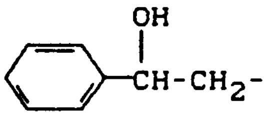 | $-CH_3$ | Fp. 92° -93° C |
| 202 | 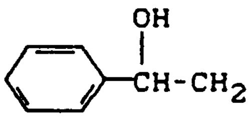 | 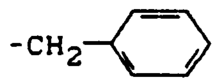 | NMR*) : 4.8 -<br>4.87<br>5.48 |
| 203 | 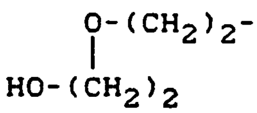 | $-CH_3$ | NMR*) : 3.7 -<br>3.8<br>4.25 |
| 204 | 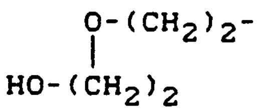 | $-C_2H_5$ | NMR*) : 3.7 -<br>3.8<br>4.5 |
| 205 | 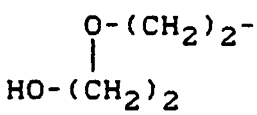 | $-(CH_2)_2-CH_3$ | NMR*) : 3.7 -<br>3.8<br>4.41 |
| 206 | 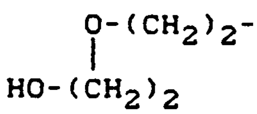 | 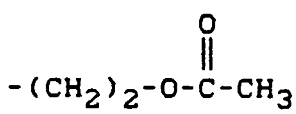 | NMR*) : 3.7 -<br>3.8<br>2.1 |
| 207 | 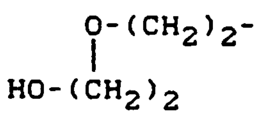 | $-CH_2-CH=CH_2$ | NMR*) : 3.7 -<br>3.8<br>4.9 |
| 208 | 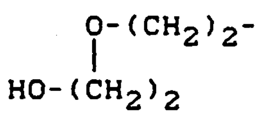 | $-CH_2-CH=CH-CH_3$ | NMR*) : 1.78<br>3.7 -<br>3.8 |

| Bsp. Nr. | $R^1$ | $R^2$ | physikalische Eigenschaften |
|---|---|---|---|
| 209 | 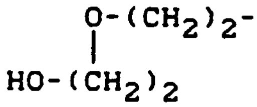 | 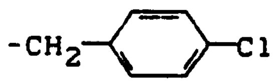 | NMR*) : 3.7-3.8<br>5.38 |
| 210 | 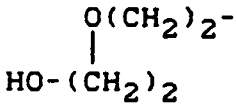 | 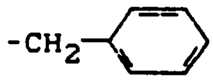 | NMR*) : 3.7-3.8<br>5.35 |
| 211 | 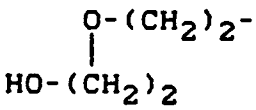 | 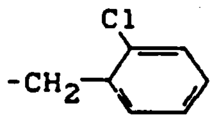 | NMR*) : 3.7-3.8<br>5.53 |
| 212 | 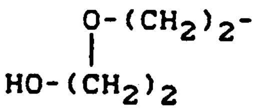 | 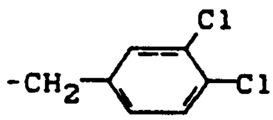 | NMR*) : 3.7-3.8<br>5.35 |
| 213 | 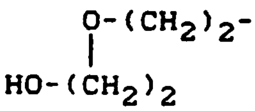 | 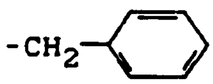 | NMR*) : 3.7-3.8<br>5.41 |
| 214 | 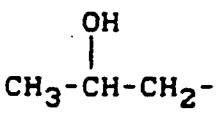 | 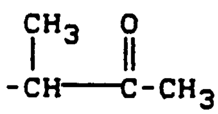 | NMR*) : 1.22<br>2.22 |
| 215 | 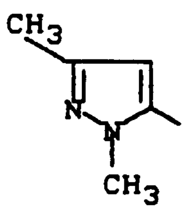 | $-CH_3$ | Fp. 116°-120° C |
| 216 | 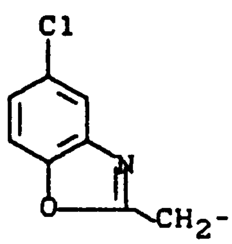 | $-CH_3$ | Fp. 129 °C |

| Bsp. Nr. | $R^1$ | $R^2$ | physikalische Eigenschaften |
|---|---|---|---|
| 217 | 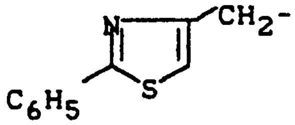 | $-CH_3$ | NMR*): 3.95<br>4.27 |
| 218 | 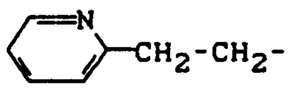 | $-CH_3$ | Fp 77° C |
| 219 | 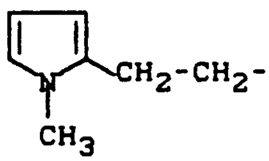 | $-CH_3$ | NMR*): 2.85<br>3.6<br>4.23 |
| 220 | 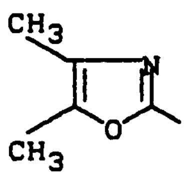 | $-CH_3$ | Fp 196° C |
| 221 | $HO-(CH_2)_3-$ | $-CH_3$ | NMR*): 3.75<br>4.28 |
| 222 | $HO-(CH_2)_2-O-(CH_2)_2-$ | $-CH_2-\overset{O}{\overset{\|}{C}}-OC_2H_5$ | $^1H$-NMR*): 4.9 |
| 223 | $HO-(CH_2)_2-O-(CH_2)_2-$ | $-\overset{CH_3}{\overset{\|}{CH}}-\overset{O}{\overset{\|}{C}}-CH_3$ | $^1H$-NMR*): 2.21 |
| 224 | $HO-(CH_2)_5-$ | $CH_3$ | $^1H$-MNR*): 4.23 |
| 225 | $HO-(CH_2)_5-$ | $C_2H_5$ | $^1H$-NMR*): 4.49 |
| 226 | $HO-(CH_2)_5-$ | $-(CH_2)_2-CH_3$ | $^1H$-NMR*): 4.38 |
| 227 | $HO-(CH_2)_5-$ | $-CH_2-CN$ | $^1H$-NMR*): 5.11 |

| Bsp. Nr. | $R^1$ | $R^2$ | physikalische Eigenschaften |
|---|---|---|---|
| 228 | $HO-(CH_2)_5-$ | $-CH_2-CH_2-O-\overset{O}{\overset{\|}{C}}-CH_3$ | $^1H$-NMR*): 2.1 |
| 229 | $HO-(CH_2)_5-$ | $-CH_2-CH=CH_2$ | $^1H$-NMR*): 4.9 |
| 230 | $HO-(CH_2)_5-$ | $-CH_2-CH=CH-CH_3$ | $^1H$-NMR*): 4.8 |
| 231 | $HO-(CH_2)_5-$ | 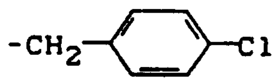 | $^1H$-NMR*): 5.38 |
| 232 | $HO-(CH_2)_5-$ | 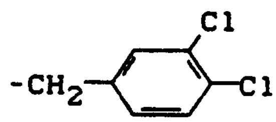 | $^1H$-NMR*): 5.35 |
| 233 | $HO-(CH_2)_5-$ | 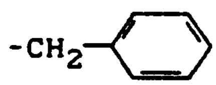 | $^1H$-NMR*): 5.4 |
| 234 | 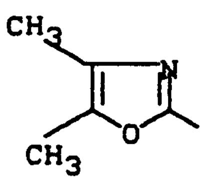 | $-CH_2-CN$ | Fp. 199° C |
| 235 | 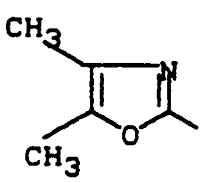 | $-CH_2-CO-CH_3$ | Fp. 169° C |
| 236 | 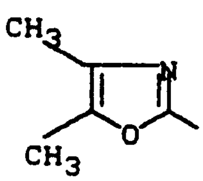 | 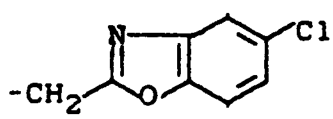 | Fp. 180° C |
| 237 | 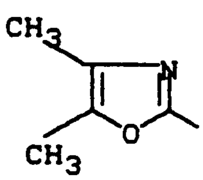 | 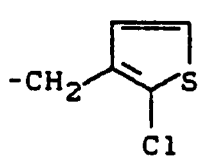 | Fp. 116° C |

| Bsp. Nr. | $R^1$ | $R^2$ | physikalische Eigenschaften |
|---|---|---|---|
| 238 | 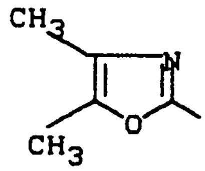 | 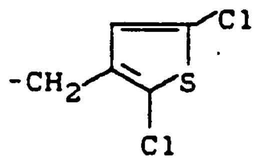 | Fp. 190° C |
| 239 | 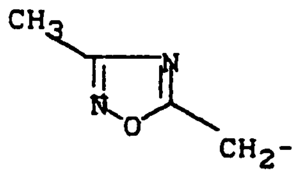 | $-CH_2-CN$ | $^1$H-NMR*): 5.2 |
| 240 | 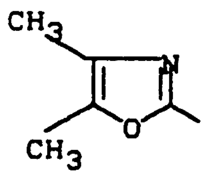 | 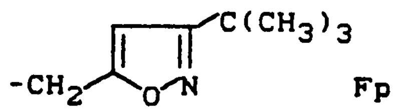 | Fp. 95° C |
| 241 | 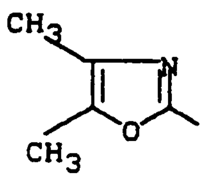 | $-CH_2-CO-OC_2H_5$ | Fp. 158° C |
| 242 | 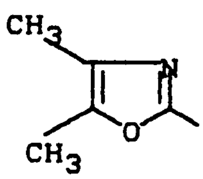 | 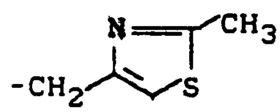 | $^1$H-NMR*): 5.5 |
| 243 | 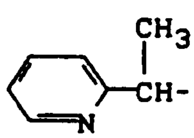 | $-CH_2CN$ | $^1$H-NMR*): 8.25 |
| 244 | 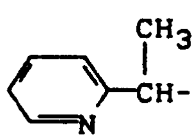 | $-CH_2-CO-OC_2H_5$ | Fp. 131° C |
| 245 | 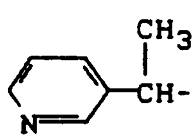 | $-CH_2-CO-OC_2H_5$ | Fp. 137° C |

| Bsp. Nr. | $R^1$ | $R^2$ | physikalische Eigenschaften |
|---|---|---|---|
| 246 | 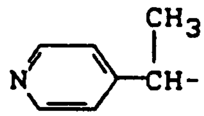 | $-CH_2-CO-CH_3$ | $^1$H-NMR*): 5.3 |
| 247 | 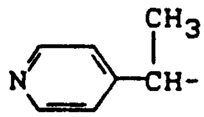 | $-CH_2-CO-OC_2H_5$ | $^1$H-NMR*): 6.8 |
| 248 | 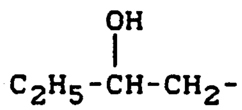 | $CH_3$ | $^1$H-NMR*): 0.98; 4.25 |
| 249 | 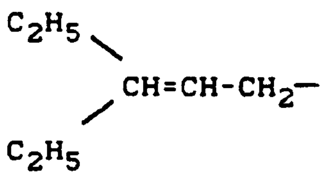 | $CH_3$ | $^1$H-NMR*): 0.92; 4.22 |

*) Die $^1$H-NMR Spektren wurden in $CDCl_3$ oder in DMSO-$d_6$ aufgenommen. Angegeben ist die chemische Verschiebung als δ-Wert in ppm.

Herstellung der Ausgangsverbindungen

Beispiel II-1:

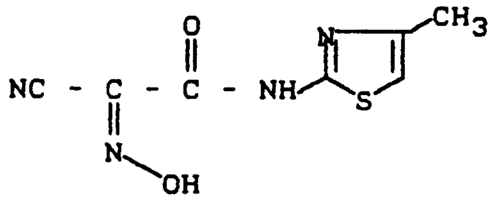

5,4g (0,03 Mol) 2-Cyanacetamido-4-methylthiazol werden in 30 ml Ethanol gelöst und mit 3,9 g (0,033 Mol) Isoamylnitrit versetzt. Danach versetzt mit 16,5 ml 2n HCl und läßt 1 Stunde bei Raumtemperatur stehen. Nach Abkühlen auf 0 °C filtriert man das Produkt ab.

Man erhält 4,9 g (77 % der Theorie) an 2-Hydroximinocyanessigsäure-N-(4-methylthiazol-2-yl)-amid vom Zersetzungspunkt 252 °C.

In entsprechender Weise und gemäß den allgemeinen Angaben zur Herstellung erhält man die folgenden 2-Cyano-2-ox-iminoacetamide der allgemeinen Formel (II):

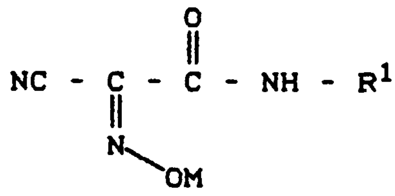 (II)

| Bsp. Nr. | $R^1$ | M | physikalische Eigenschaften |
|---|---|---|---|
| II-2 | 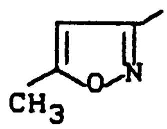 | H | Zersetzungspunkt 238 °C |
| II-3 | 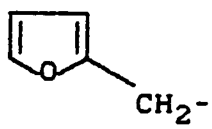 | H | Fp. 178-180° C |

Anwendungsbeispiele:

In den folgenden Anwendungsbeispielen wurde die nachstehend aufgeführte Verbindung als Vergleichssubstanz eingesetzt:

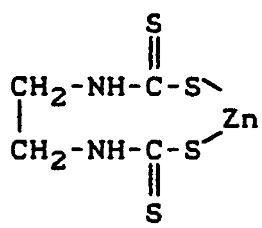

Zink-ethylen-1,2-bis-dithiocarbamat

Beispiel A

Phytophthora-Test (Tomate) /kurativ

Lösungsmittel: 4,7 Gewichtsteile Aceton
Emulgator: 0,3 Gewichtsteile Alkyl-aryl-poloyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20 °C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100% relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen:
2, 4, 5, 6, 7, 11, 12, 13, 27, 28, 29, 30, 32, 33, 34, 120.

## Patentansprüche

**1.** 2-Cyano-2-alkoximino-acetamide der allgemeinen Formel (I),

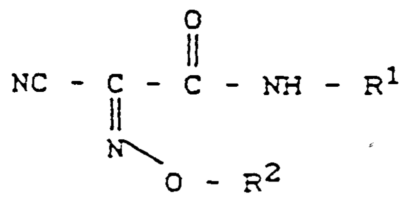
(I)

in welcher

$R^1$ für jeweils geradkettiges oder verzweigtes, gegebenenfalls durch Phenyl substituiertes Hydroxyalkyl oder Hydroxyalkoxyalkyl mit jeweils 1 bis 4 Kohlenstoffatomen in den einzelnen Alkylteilen steht, außerdem für jeweils gegebenenfalls ein- bis dreifach, gleich oder verschieden im Arylteil substituiertes Heteroaryl oder Heteroarylalkyl mit 1 bis 4 Kohlenstoffatomen im geradkettigen oder verzweigten Alkylteil steht, wobei als Heteroarylrest jeweils vorkommen kann: Pyridyl, Pyrimidyl, Triazinyl, Chinolyl, Isochinolyl, Oxazolyl, Isoxazolyl, Oxadiazolyl, Benzoxazolyl, Thiazolyl, Isothiazolyl, Thiadiazolyl, Benzthiazolyl, Imidazolyl, Benzimidazolyl, Pyrrolyl, Furanyl, Thienyl, Pyrazolyl oder Triazolyl und wobei als Substituenten im Heteroarylteil oder im benzannellierten Ring jeweils in Frage kommen: Hydroxy, Fluor, Chlor, Brom, Cyano, Methyl, Ethyl, n- oder i-Propyl, n-, i-, s- oder t-Butyl, Allyl, Butenyl, Methoxy, Ethoxy, Methylthio, Benzyl, Phenyl, Acetyl, Propionyl, Methoxycarbonyl, Ethoxycarbonyl, Methylaminocarbonyl oder Dimethylaminocarbonyl und

$R^2$ für gegebenenfalls ein- bis dreifach, gleich oder verschieden substituiertes, geradkettiges oder verzweigtes Alkyl mit 1 bis 12 Kohlenstoffatomen steht, wobei als Substituenten genannt seien: Cyano, Acetyl, Propionyl, Methoxycarbonyl, Ethoxycarbonyl, Methylcarbonyloxy, Ethylcarbonyloxy, gegebenenfalls ein- bis dreifach, gleich oder verschieden durch Fluor, Chlor, Brom, Methyl und/oder Ethyl substituiertes Phenyl; außerdem für jeweils geradkettiges oder verzweigtes Alkenyl oder Halogenalkenyl mit 3 bis 6 Kohlenstoffatomen und gegebenenfalls 1 bis 3 Halogenatomen oder für geradkettiges oder verzweigtes Alkinyl mit 3 bis 6 Kohlenstoffatomen steht, ausgenommen die Verbindungen 2-(2-Cyano-2-methoximino-acetamido, thiazol, 3-Methyl-5-(2-cyano-2-methoximino-acetamido)-isooxazol und 2-Methyl-5-(2-cyano-2-methoximino-acetamido)-1,3,4-thiadiazol.

**2.** Verfahren zur Herstellung von 2-Cyano-2-alkoximino-acetamide der allgemeinen Formel (I),

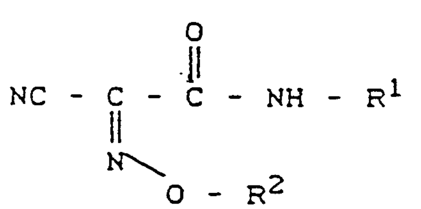
(I)

in welcher

$R^1$ und $R^2$ die in Anspruch 1 angegebene Bedeutung haben,

daß man 2-Cyano-2-oximino-acetamide der Formel (II),

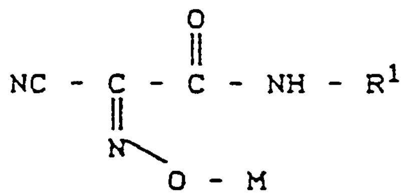

(II)

in welcher
M für Wasserstoff oder für ein Alkalimetallkation steht und
$R^1$ die oben angegebene Bedeutung hat,
mit Alkylierungsmitteln der Formel (III),

$R^2$ - A (III)

in welcher
A für eine geeignete Abgangsgruppe steht und
$R^2$ die oben angegebene Bedeutung hat,
gegebenenfalls in Gegenwart eines Verdünnungsmittels und gegebenenfalls in Gegenwart eines Säurebindemittels umsetzt.

**3.** Schädlingsbekämpfungsmittel, gekennzeichnet durch einen Gehalt an mindestens einer Verbindung der allgemeinen Formel (I) gemäß Anspruch 1.

**4.** Verfahren zur Bekämpfung von Schädlingen, dadurch gekennzeichnet, daß man mindestens eine Verbindung der Formel (I) gemäß Anspruch 1 auf Schädlinge und/oder ihren Lebensraum einwirken läßt.

**5.** Verwendung von Verbindungen der allgemeinen Formel (I) gemäß Anspruch 1 zur Bekämpfung von Schädlingen.

**6.** Verfahren zur Herstellung von Schädlingsbekämpfungsmitteln, dadurch gekennzeichnet, daß man Verbindungen der allgemeinen Formel (I) gemäß Anspruch 1 mit Streckmitteln und/oder oberflächenaktiven Mitteln vermischt.

**7.** 2-Cyano-2-oximino-acetamide der allgemeinen Formel (II),

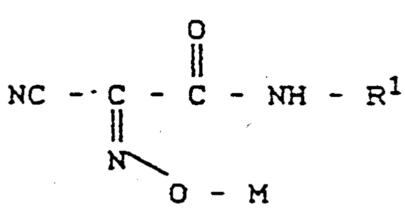

(II)

in welcher
M für Wasserstoff oder für ein Alkalimetallkation steht und
$R^1$ die in Anspruch 1 angegebene Bedeutung hat.

## Claims

**1.** 2-Cyano-2-alkoximino-acetamides of the general formula (I),

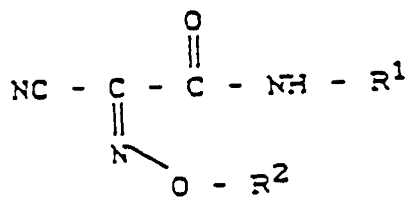

(I)

in which

R[1] represents in each case straight-chain or branched, optionally phenyl-substituted hydroxyalkyl or hydroxyalkoxyalkyl in each case having 1 to 4 carbon atoms in the individual alkyl parts, or, additionally, represents heteroaryl or heteroarylalkyl, having 1 to 4 carbon atoms in the straight-chain or branched alkyl part, which is in each case optionally monosubstituted, disubstituted or trisubstituted in the aryl part by identical or different substituents and it being possible for the heteroaryl radical to be the following in each case: pyridyl, pyrimidyl, triazinyl, quinolyl, isoquinolyl, oxazolyl, isoxazolyl, oxadiazolyl, benzoxazolyl, thiazolyl, isothiazolyl, thiadizolyl, benzothiazolyl, imidazolyl, benzimidazolyl, pyrrolyl, furanyl, thienyl, pryazolyl or triazolyl and the following being suitable in each case as substituents in the heteroaryl part or in the benzo-fused ring: hydroxyl, fluorine, chlorine, bromine, cyano, methyl, ethyl, n- or i-propyl, n-, i-, s- or t-butyl, allyl, butenyl, methoxy, ethoxy, methylthio, benzyl, phenyl, acetyl, propionyl, methoxycarbonyl, ethoxycarbonyl, methylaminocarbonyl or dimethylaminocarbonyl, and

R[2] represents straight-chain or branched alkyl, having 1 to 12 carbon atoms, which is optionally monosubstituted, disubstituted or trisubstituted, the substituents being identical or different and substituents which may be mentioned being: cyano, acetyl, propionyl, methoxycarbonyl, ethoxycarbonyl, methylcarbonyloxy, ethylcarbonyloxy, or phenyl which is optionally monosubstituted, disubstituted or trisubstituted by identical or different substituents from the series consisting of fluorine, chlorine, bromine, methyl and/or ethyl; in addition represents in each case straight-chain or branched alkenyl or halogenoalkenyl having 3 to 6 carbon atoms and, if appropriate, 1 to 3 halogen atoms, or straight-chain or branched alkinyl having 3 to 6 carbon atoms, except for the compounds 2-(2-cyano-2-methoximino-acetamido)-thiazole, 3-methyl-5-(2-cyano-2-methoximino-acetamido)-isoxazole and 2-methyl-5-(2-cyano-2-methoximino-acetamido)-1,3,4-thiadiazole.

**2.** Process for the preparation of 2-cyano-2-alkoximinoacetamides of the general formula (I),

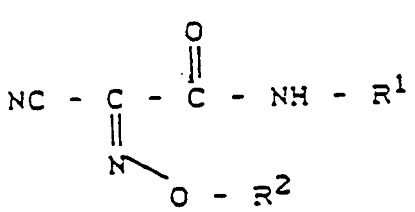

(I)

in which

$R^1$ and $R^2$ have the meaning specified in Claim 1, characterised in that 2-cyano-2-oximino-acetamides of the formula (II),

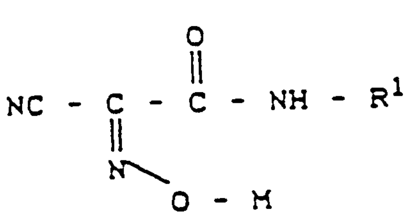

(II)

in which

M represents hydrogen or an alkali metal cation, and

$R^1$ has the abovementioned meaning,

are reacted with alkylating agents of the formula (III)

$R^2$ - A (III)

in which

A represents a suitable leaving group, and

$R^2$ has the abovementioned meaning,

if appropriate in the presence of a diluent and if appropriate in the presence of an acid-binding agent.

**3.** Pesticides, characterised in that they contain at least one compound of the general formula (I) according to Claim 1.

**4.** Process for combating pests, characterised in that at least one compound of the formula (I) according to Claim 1 is allowed to act on pests and/or their habitat.

**5.** Use of compounds of the general formula (I) according to Claim 1 for combating pests.

**6.** Process for the preparation of pesticides, characterised in that compounds of the general formula (I) according to Claim 1 are mixed with extenders and/or surface-active agents.

**7.** 2-Cyano-2-oximino-acetamides of the general formula (II),

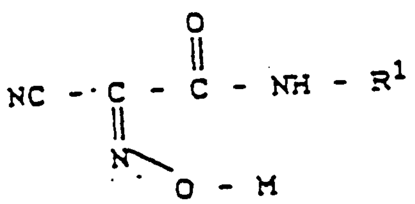

(II)

in which

M represents hydrogen or an alkali metal cation, and

$R^1$ has the meaning specified in Claim 1.

**Revendications**

**1.** 2-cyano-2-alcoximino-acétamides de formule générale I

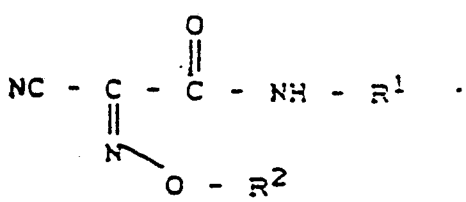

(I)

dans laquelle

$R^1$ représente un groupe hydroxyalkyle ou hydroxyalcoxyalkyle à chaîne droite ou ramifiée éventuellement substitué par un groupe phényle, contenant chacun 1 à 4 atomes de carbone dans les diverses parties alkyle, un groupe hétéroaryle ou hétéarylalkyle contenant 1 à 4 atomes de carbone dans la partie alkyle à chaîne droite ou ramifiée, et portant éventuellement 1 à 3 substituants identiques ou différents dans la partie aryle, le groupe hétéroaryle pouvant être choisi parmi les suivants : pyridyle, pyrimidyle, triazinyle, quinoléyle, isoquino-

léyle, oxazolyle, isoxazolyle, oxadiazolyle, benzoxazolyle, thiazolyle, isothiazolyle, thiadiazolyle, benzothiazolyle, imidazolyle, benzimidazolyle, pyrrolyle, furannyle, thiényle, pyrazolyle ou triazolyle, et les substituants de la partie hétéroaryle ou du noyau condensé sur le noyau benzénique consistant en : des groupes hydroxy, le fluor, le chlore, le brome, des groupes cyano, méthyle, éthyle, n- ou iso-propyle, n-, iso-, sec-, tert-butyle, allyle, butényle, méthoxy, éthoxy, méthylthio, benzyle, phényle, acétyle, propionyle, méthoxycarbonyle, éthoxycarbonyle, méthylaminocarbonyle ou diméthylaminocarbonyle, et

$R^2$ représente un groupe alkyle à chaîne droite ou ramifiée en $C_1$-$C_{12}$ portant éventuellement 1 à 3 substituants identiques ou différents, ces substituants étant choisis parmi les groupes cyano, acétyle, propionyle, méthoxycaronyle, éthoxycarbonyle, méthylcarbonyloxy, éthylcarbonyloxy, phényle portant éventuellele fluor, te chlore, le brome, les groupes méthyle et/ou éthyle ; un groupe alcényle ou halognéoalcényle à chaîne droite ou ramifiée contenant 3 à 6 atomes de carbone et le cas échéant 1 à 3 atomes d'halogènes, ou un groupe alcynyle à chaîne droite ou ramifiée en $C_3$-$C_6$,

à l'exception des composés : 2-(2-cyano-2-méthoximino-acétamido)-thiazole, 3-méthyl-5-(2-cyano-2-méthoximino-acétamido) isoxazole et 2-méthyl-5-(2-cyano-2-méthoximino-acétamido)-1,3,4-tiadiazole.

**2.** Procédé de préparation des 2-cyano-2-alcoximino-acétamides de formule générale I

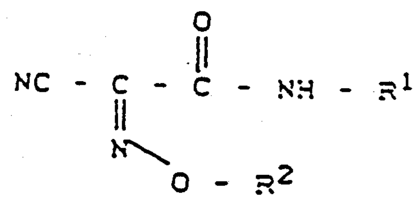

dans laquelle $R^1$ et $R^2$ ont les significations indiquées dans la revendication 1, caractérisé en ce que l'on fait réagir des 2-cyano-2-oximino-acétamides de formule II

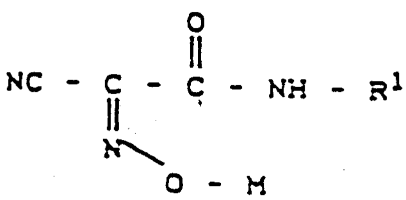 (II)

dans laquelle
M représente l'hydrogène ou un cation de métal alcalin, et
$R^1$ a les significations indiquées ci-dessus,
avec des agents alkylants de formule III

$R^2$-A (III)

dans laquelle
A représente un groupe éliminable approprié, et
$R^2$ a les significations indiquées ci-dessus,
éventuellement en présence d'un diluant et éventuellement en présence d'un accepteur d'acide.

**3.** Produit pesticide caractérisé en ce qu'il contient au moins un composé de formule générale I de la revendication 1.

**4.** Procédé pour combattre les parasites, caractérisé en ce que l'on fait agir au moins un composé de formule I de la revendication 1 sur les parasites et/ou leur habitat.

5. Utilisation de composés de formule générale I de la revendication 1 pour la lutte contre les parasites.

6. Procédé de préparation de produits pesticides, caractérisé en ce que l'on mélange des composés de formule générale I de la revendication 1 avec des diluants et/ou des agents tensioactifs.

7. 2-cyano-2-oximino-acétamides de formule générale II

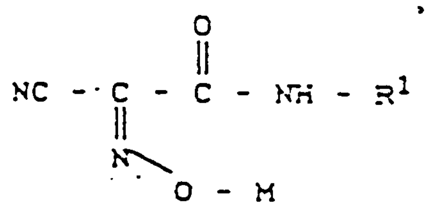

(II)

dans laquelle
M représente l'hydrogène ou un cation de métal alcalin, et
$R^1$ a les significations indiquées dans la revendication 1.